(19) Europäisches Patentamt / European Patent Office / Office européen des brevets

(11) **EP 2 896 635 B1**

(12) **EUROPEAN PATENT SPECIFICATION**

(45) Date of publication and mention
of the grant of the patent:
**23.05.2018 Bulletin 2018/21**

(51) Int Cl.:
**C08F 220/18** (2006.01)    **A61B 1/005** (2006.01)
**C08G 18/62** (2006.01)     **C09D 133/06** (2006.01)
**C09D 175/04** (2006.01)

(21) Application number: **14197419.6**

(22) Date of filing: **11.12.2014**

(54) **Flexible tube, endoscopic medical apparatus, and resin composition for top coat layer**

Beweglicher Schlauch, medizinische Endoskopvorrichtung und Harzzusammensetzung für Deckschicht

Tube flexible, appareil médical endoscopique et composition de résine pour couche de revêtement supérieure

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.01.2014 JP 2014007308**

(43) Date of publication of application:
**22.07.2015 Bulletin 2015/30**

(73) Proprietor: **Fujifilm Corporation**
**Minato-ku**
**Tokyo 106-8620 (JP)**

(72) Inventor: **Watanabe, Joji**
**Ashigarakami-gun**
**Kanagawa, 258-8538 (JP)**

(74) Representative: **Klunker IP**
**Patentanwälte PartG mbB**
**Destouchesstraße 68**
**80796 München (DE)**

(56) References cited:
**EP-A2- 2 168 471         WO-A1-2013/056815**
**US-A1- 2009 069 631      US-A1- 2010 249 315**

**Description**

**BACKGROUND OF THE INVENTION**

1. Field of the Invention

The present invention relates to a flexible tube, and an endoscopic medical apparatus. A resin composition for top coat layer is also described.

2. Description of the Related Art

An endoscope is a medical apparatus for observing the inside of the body cavity of a patient. Because the endoscope is used by being inserted into the body cavity, the apparatus is required not to damage organs and not to cause a patient to feel pain or discomfort. In order to meet such a requirement, for a flexible tube constituting an insertion portion of the endoscope, a spiral tube formed by winding a flexibly bendable metal strip in the form of a spiral is adopted. Furthermore, in order to prevent the surface of the esophagus, bowel, or the like from being irritated or damaged, a method of covering the periphery of the spiral tube with a flexible resin and then further covering the resultant with a topcoat layer has been devised.

Examples of an acrylic resin constituting the topcoat layer include resins using acrylamide (JP2010-239981A and JP2010-503436A). According to JP2010-239981A and JP2010-503436A, the use of such resins makes it possible to realize excellent performance in regards to adhesiveness, chemical resistance, and abrasion resistance.

Furthermore, JP1995-039511A (JP-H07-039511A) proposes a top coat composition prepared by using a fluoroolefin polymer and hexamethylene diisocyanate as a curing agent. According to JP1995-039511 A (JP-H07-039511A), the use of such a composition makes it possible to accomplish both flexibility and low frictional resistance.

In addition, for example, JP3652138B describes using a silica coating layer converted from perhydropolysilazane, JP3542208B describes performing chemical vapor deposition of poly-para-xylene, JP3500219B describes a case of using an aliphatic prepolymer and a friction reducer. EP21684714 discloses an endoscope comprising a flexible tube constituted of a flexible tubular structure, a jacket for covering the periphery of the structure, and a coating layer for coating the jacket wherein the coating layer is made of a fluorocarbon polymer, silicone resin or polyurethane resin, and imparts resistance to a chemical agent such as disinfectant. US 2009/069631 discloses flexible tubes for endoscopes, which has a surface coated with an outer covering, wherein the outer covering is formed of a material comprising a polyolefin thermoplastic elastomer.

**SUMMARY OF THE INVENTION**

As described above, regarding resins forming the top coat layer of endoscopes and other diagnostic instruments for use in body cavities, sufficient research and development have not yet been performed, and currently, various resins or fixing methods are being tested by trial and error.

In this situation, in order to reduce the strain that patients suffer from, the present inventor has sought to maintain the performance of the endoscope at a high level while improving sliding properties thereof. In contrast, in the technique of JP1995-039511A (JP-H07-039511A) applied by the present applicant, flexibility of the resin could be further improved, hence the inventor focused particularly on the development of a technique that can further improve bending resilience of an insertion portion of a medical instrument. In the related art, in order to secure the resilience of the insertion portion, means for using a urethane elastomer as a material of a molding resin layer positioned under a top coat layer has been used. However, when chemical resistance or abrasion resistance was regarded as being important, the thickness of the topcoat layer had to be increased, and accordingly, resilience of the molding resin layer was reduced.

In this regard, the claimed invention aims to provide a flexible tube that is used for an endoscope has a high degree of chemical resistance, abrasion resistance, or sliding properties that are realized by the improvement of a top coat layer thereof, and has a covering layer exhibiting excellent resilience; an endoscopic medical apparatus that uses such flexible tube; and a resin composition for top coat layer that is used for such flexible tube.

The above object was achieved by the following means.

[1] A flexible tube including: a flexible tube substrate that has a cylindrical shape and flexibility; a resin layer that covers the flexible tube substrate; and a top coat layer that covers the resin layer, in which the top coat layer contains a polymer compound I derived from monomers including the following A to C,

Monomer A: hydroxyl group-free acrylate having a molecular weight equal to or
greater than 110                                                                                                   100 parts by mass,

(continued)

| Monomer B: hydroxyl group-free acrylate having a molecular weight equal to or greater than 80 and less than 110 | 10 parts by mass to 200 parts by mass, |
| Monomer C: hydroxyalkyl acrylate | 1 part by mass to 60 parts by mass. |

[2] The flexible tube described in [1], in which the monomer A is butyl acrylate or hexyl acrylate.

[3] The flexible tube described in [1] or [2], in which the monomer B is ethyl acrylate, methyl acrylate, or methyl methacrylate.

[4] The flexible tube described in any one of [1] to [3], in which the monomer C is hydroxyethyl acrylate, hydroxymethyl acrylate, hydroxypropyl acrylate, or hydroxybutyl acrylate.

[5] The flexible tube described in any one of [1] to [4], in which the resin layer contains at least one kind selected from among a urethane elastomer, a polyurethane elastomer, and a polyamide elastomer.

[6] The flexible tube described in any one of [1] to [5], in which the top coat layer is a layer formed by curing the polymer compound I with a curing agent.

[7] The flexible tube described in any one of [1] to [6], in which a storage modulus $E_T'$ of the material of the top coat layer is smaller than a storage modulus $E_R'$ of the material of the resin layer.

[8] The flexible tube described in [7], in which the storage modulus $E_T'$ of the material of the top coat layer is equal to or greater than 1 MPa and equal to or less than 20 MPa.

[9] The flexible tube described in [7] or [8], in which the storage modulus $E_R'$ of the material of the resin layer is equal to or greater than 1 MPa and equal to or less than 500 MPa.

[10] The flexible tube described in [7] or [8], in which $E_R' - E_T'$, which represents a difference between the storage modulus $E_T'$ of the material of the top coat layer and the storage modulus $E_R'$ of the material of the resin layer, is equal to or greater than 1 MPa and equal to or less than 200 MPa.

[11] The flexible tube described in any one of [1] to [10], in which a thickness of the top coat layer is equal to or greater than 10 $\mu$m and equal to or less than 200 $\mu$m.

[12] The flexible tube described in any one of [1] to [11], in which the resin layer has an inner layer and an outer layer, and a thickness ratio between the inner layer and the outer layer changes with a gradient in an axial direction of the flexible tube substrate.

[13] The flexible tube described in any one of [1] to [12], in which the resin layer has an inner layer and an outer layer, a storage modulus of the inner layer is equal to or greater than 1 MPa and equal to or less than 150 MPa, a storage modulus of the outer layer is equal to or greater than 1 MPa and equal to or less than 500 MPa, and a difference obtained by subtracting the storage modulus of the inner layer from the storage modulus of the outer layer is equal to or greater than 20 MPa and equal to or less than 200 MPa.

[14] The flexible tube described in [12], in which in a first end portion of the flexible tube substrate, the thickness ratio between the inner layer and the outer layer is 5:95 to 40:60 (inner layer:outer layer), in a second end portion of the flexible tube substrate, the thickness ratio is 95:5 to 60:40 (inner layer:outer layer), and inversion of the thickness ratio occurs between the first end portion and the second end portion.

[15] The flexible tube described in any one of [1] to [14] in which the flexible tube is configured to be used for an endoscopic medical apparatus.

[16] An endoscopic medical apparatus including the flexible tube described in any one of [1] to [15].Thus, a resin composition for top coat layer that is for forming the top coat layer covering the flexible tube, comprises the following monomers A to C,

| Monomer A: hydroxyl group-free acrylate having a molecular weight equal to or greater than 110 | 100 parts by mass, |
| Monomer B: hydroxyl group-free acrylate having a molecular weight equal to or greater than 80 and less than 110 | 10 parts by mass to 200 parts by mass, |
| Monomer C: hydroxyalkyl acrylate | 1 part by mass to 60 parts by mass. |

The resin composition described above may further comprise a curing agent.

In the present specification, when there is a plurality of substituents, linking groups, marked with a specific sign, when the plurality of substituents are specified simultaneously, or when one of the plurality of substituents is specified selectively, it means that the respective substituents may be the same as or different from each other. Moreover, unless otherwise specified, when the plurality of substituents are adjacent to each other, they may form a ring by being linked to each other or by causing ring condensation.

In the present specification, when there is no description regarding whether or not a substituent (linking group) is substituted, the substituent (linking group) may have any substituent as long as an intended effect is obtained. Similarly, when there is no description regarding whether or not a compound is substituted, the compound may have any substituent.

In the present specification, in a broad sense, the term "acrylate" is used as a generic term of ester compounds having a substituted or unsubstituted acryloyl group ($-C=O-C(R)=CH_2$: R is a halogen atom, an alkyl group having 1 to 3 carbon atoms, a hydroxy group, a halogen atom, or a cyano group). In a narrow sense, it means a compound having an acryloyl group (R=H). An acrylate compound according to the claimed invention is preferably acrylate (R=H) or methacrylate ($R=CH_3$).

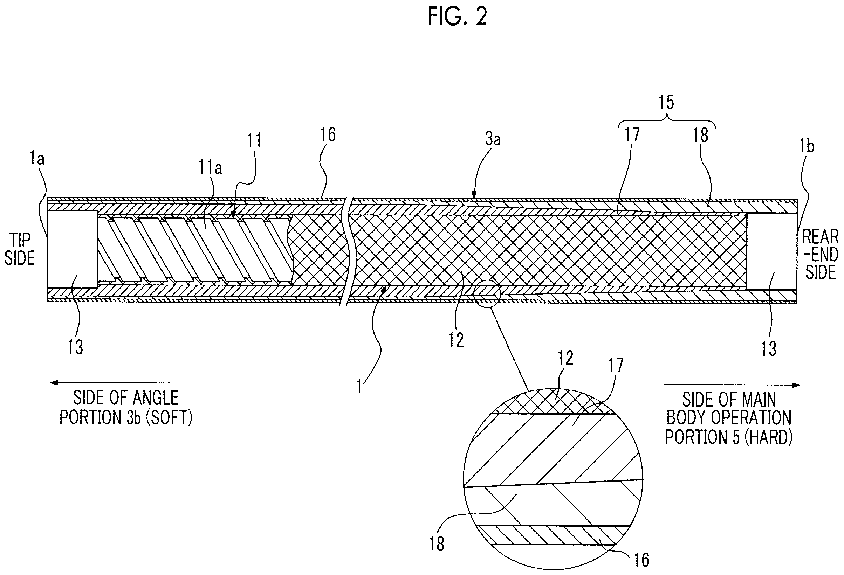

In the present specification, the directions in the flexible tube includes two directions, one direction being a direction heading toward the inside of the flexible tube and the other being a direction heading toward the outside of the flexible tube. Hereinafter, the direction heading toward the inside of the flexible tube is referred to as an inner side or a lower side, and the direction heading toward the outside is referred to as an outer side or an upper side. For example, in the embodiment of Fig. 2, a top coat layer 16 is at the outer side (upper side) of a resin layer 15.

With the flexible tube according to the claimed invention and an endoscopic medical apparatus using the flexible tube, when such an apparatus is used for making a diagnosis in body cavities, a high degree of chemical resistance, abrasion resistance, and sliding properties of the flexible tube are realized, and the covering layer exhibits excellent resilience. Accordingly, in a medical diagnosis, excellent durability of the apparatus, excellent operability for a physician, and reduction in strain on a patient can be satisfied.

## BRIEF DESCRIPTION OF THE DRAWINGS

Fig. 1 is an external view showing the configuration of an electronic endoscope.
Fig. 2 is a partial cross-sectional view showing the schematic configuration of a flexible tube.
Fig. 3 is a lateral view showing a sliding tester used in examples.
Fig. 4 is a lateral view schematically showing a Tensilon apparatus used in examples.

## DESCRIPTION OF THE PREFERRED EMBODIMENTS

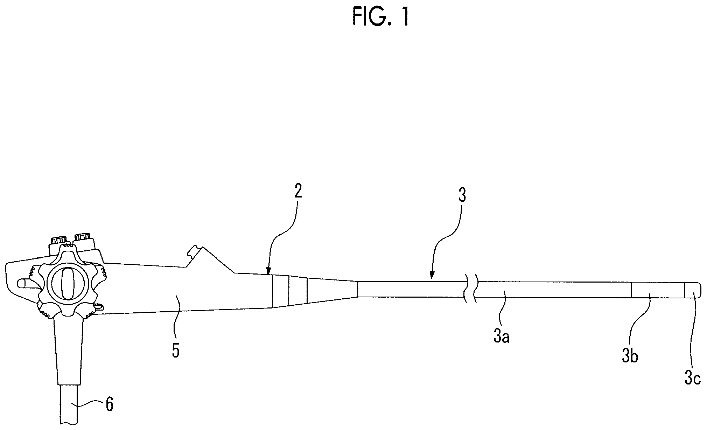

An electronic endoscope according to a preferable embodiment of the present invention is equipped with a flexible tube. Such product is widely used for medical purposes. In the example shown in Fig. 1, an electronic endoscope 2 includes an insertion portion 3 that is inserted into a body cavity; a main body operation portion 5 that is connected to the base portion of the insertion portion 3; and a universal cord 6 that is connected to a processor apparatus or a light source apparatus. The insertion portion 3 is constituted by a flexible tube 3a that is connected to the main body operation portion 5; an angle portion 3b that is connected to the flexible tube 3a; and a tip portion 3c that is connected to the tip of the angle portion 3b and has a built-in imaging device (not shown in the drawing) for taking images in a body cavity. The flexible tube 3a occupying most of the insertion portion 3 exhibits flexibility approximately over the full length thereof, and a region of the tube that is inserted into a body cavity has a structure particularly excellent in flexibility.

(Flexible tube)

As shown in Fig. 2, the flexible tube 3a (flexible tube for endoscope) has a configuration in which the outer peripheral surface of a flexible tube substrate 1 is covered with a resin layer 15. The flexible tube substrate 1 has a spiral tube 11, which is formed by winding a metal strip 11a in the form of a spiral, at the innermost side thereof, and a cylindrical mesh 12 covering the spiral tube 11. The cylindrical mesh 12 is formed by braiding metal wires. The flexible tube substrate 1 also has ferrules 13 that are fitted to both ends of the spiral tube 11 covered with the cylindrical mesh 12. Furthermore, in the flexible tube 3a of the present embodiment, the outer surface of the resin layer 15 is coated with the top coat layer 16 having chemical resistance. Although Fig. 2 shows the spiral tube 11 constituted by a single layer, the spiral tube 11 may be constituted by two layers concentrically disposed on each other. The resin layer 15 and the top coat layer 16 are collectively called a covering layer in some cases. In Fig. 2, the resin layer 15 and the top coat layer 16 are illustrated as thick layers relative to the diameter of the flexible tube substrate 1, such that the layer structure thereof can be clearly understood.

The resin layer 15 according to the present embodiment covers the outer peripheral surface of the flexible tube substrate 1. The resin layer 15 has a double layered structure as a laminate consisting of an inner layer 17, which covers the entire peripheral surface of the flexible tube substrate 1 around the axis thereof, and an outer layer 18, which covers the entire peripheral surface of the inner layer 17 around the axis thereof. A soft resin is used as the material of the inner layer 17, and a hard resin is used as the material of the outer layer 18.

In the present embodiment, the resin layer 15 is formed such that it has approximately a uniform thickness in the longitudinal direction (axial direction) of the flexible tube substrate 1. The thickness of the resin layer 15 is, for example, 0.2 mm to 1 mm, and an outer diameter D of the flexible tube 3a is, for example, 11 mm to 14 mm. The inner layer 17 and the outer layer 18 are formed such that a ratio of the thickness of each of the inner layer 17 and the outer layer 18 to the total thickness of the resin layer 15 changes in the axial direction of the flexible tube substrate 1. Specifically, at the side (tip side) of a first end portion 1a of the flexible tube substrate 1 mounted on the angle portion 3b, the ratio of the thickness of the inner layer 17 to the total thickness of the resin layer 15 is greater than the ratio of the thickness of the outer layer 18 to the total thickness of the resin layer 15. Then the thickness of the inner layer 17 gradually decreases from the first end portion 1a toward the side (base side) of a second end portion 1b mounted on the main body operation portion 5, and at the side of the second end portion 1b, the outer layer 18 becomes thicker than the inner layer 17.

At both ends 1a and 1b of the present embodiment, the thickness ratio of each of the inner layer 17 and the outer layer 18 maximized. In the first end portion 1a, the thickness ratio between the inner layer 17 and the outer layer 18 is 9:1, and in the second end portion 1b, the thickness ratio between the inner layer 17 and the outer layer 18 is 1:9. In this way, the thickness ratio between the inner layer 17 and the outer layer 18 changes such that it undergoes inversion between the both ends 1a and 1b. Consequentially, in the flexible tube 3a, there is a difference in hardness between the side of the first end portion 1a and the side of the second end portion 1b, and flexibility of the flexible tube 3a changes in the axial direction thereof such that the side of the first end portion 1a becomes soft, and the side of the second end portion 1b becomes hard. The thickness ratio between the inner layer and the outer layer is preferably within a range of 5:95 to 40:60 (inner layer:outer layer) in the first end portion 1a, and preferably within a range of 95:5 to 60:40 (inner layer:outer layer) in the second end portion 1b. The thickness ratio between the inner layer 17 and the outer layer 18 is preferably within a range of 5:95 to 95:5 as described in the above example. The thickness ratio within this range also makes it possible to more accurately control the amount of a resin extruded into the thin layer.

A difference in a value of 100% modulus, which is an index showing hardness after molding, between the soft resin and the hard resin used for the inner layer 17 and the outer layer 18 is preferably equal to or greater than 1 MPa, and more preferably equal to or greater than 3 MPa. A difference in melt viscosity at a molding temperature of 150°C to 300°C, which is an index showing the fluidity of a molten resin, between the soft resin and the hard resin is preferably equal to or less than 2,500 PaS. Upon these conditions, in the resin layer 15 consisting of the inner layer 17 and the outer layer 18, both excellent molding accuracy and difference in hardness required between the tip side and the base side are secured.

(Method for manufacturing flexible tube)

Hereinafter, an example of a method for manufacturing the flexible tube 3a, in which the resin layer 15 has a double layered structure consisting of the inner layer 17 and the outer layer 18, will be described. Here, even when an embodiment in which the resin layer 15 consists of a single layer or three or more layers is adopted, the flexible tube 3a can be manufactured based on the following method.

In order to form the resin layer 15 constituted by at least two layers including the inner layer 17 and the outer layer 18, it is preferable to (i) prepare a first resin layer constituting the inner layer 17, (ii) prepare a second resin layer constituting the outer layer 18, and (iii) cover the flexible tube substrate 1 with the resin layer 15 by extrusion-molding the first resin material and the second resin material onto the periphery of the flexible tube substrate 1 by means of melting and kneading.

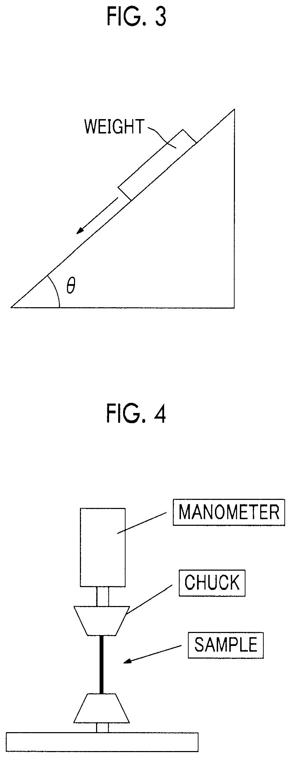

In brief, it is preferable to use a continuous molding machine for forming the resin layer 15 in the method for manufacturing the flexible tube 3a. As the continuous molding machine, for example, it is preferable to use a continuous molding machine including an extrusion portion that is constituted by a hopper, a screw, and the like; a head portion that is for molding the resin layer 15 covering the outer peripheral surface of the flexible tube substrate; a cooling portion; a transport portion (consisting of a supply drum and a winding drum) that transports a connected flexible tube substrate connected thereto to the head portion; and a control portion that controls the aforementioned portions. The head portion preferably consists of a nipple, a dice, and a supporter which supports these such that they become immobile. Such an apparatus can be constituted by, for example, the apparatus described in Figs. 3 to 5 of JP2011-72391A.

The molding temperature is preferably set within a range of 150°C to 300°C. Regarding the process performed for molding the resin layer 15 on the connected flexible tube substrate by using the continuous molding machine, when the continuous molding machine performs a molding process, the molten soft resin and the molten hard resin are extruded into the head portion from the extrusion portion. At the same time, the transport portion operates such that the connected flexible tube substrate is transported to the head portion. At this time, the soft resin and the hard resin are constantly extruded from the extrusion portion and are supplied to the head portion. The soft resin and the hard resin, which have been extruded toward a gate from the extrusion portion, pass through an edge and join together. Thereafter, in a state of being layered on each other, the resins pass through a resin path and are supplied to a molding path. As a result, the double layered resin layer 15 is molded in which the outer layer 18 using the hard resin is superposed on the inner layer 17 using the soft resin.

The connected flexible tube substrate consists of a plurality of flexible tube substrates 1 connected to each other. While the connected flexible tube substrate is being transported through the molding path, the resin layer 15 is continuously molded for the plurality of flexible tube substrates 1. When the resin layer 15 is molded for a single flexible tube substrate 1 from the side (tip side) of the first end portion 1a to the side (base side) of the second end portion 1b, at a point in time when the resin has just been extruded from the extrusion portion, the inner layer 17 has a great thickness. Thereafter, in the middle portion heading toward the side of the second end portion 1b, the thickness ratio of the outer layer 18 is gradually reduced. In this way, it is preferable to control the amount of the resin extruded such that the thickness ratio of the resin layer 15 has a gradient as described above.

A joint member is a connection portion between two flexible tube substrates 1. Accordingly, the joint member is used for switching the amount of the resin extruded from the extrusion portion controlled by the control portion. Specifically, the control portion preferably switches the amount of the resin extruded from the extrusion portion, such that the thickness ratio at the side (base side) of the first end portion 1a of one flexible tube substrate 1 is changed to the thickness ratio at the side (tip side) of the second end portion 1b of the next flexible tube substrate 1. Likewise, when the resin layer 15 is molded for the next flexible tube substrate 1 from the side of the first end portion 1a to the side of the second end portion 1b, it is preferable for the extrusion portion to be controlled, such that the thickness of the outer layer 18 gradually increases toward the side of the second end portion 1b from the side of the first end portion 1a.

The connected flexible tube substrate, in which the resin layer 15 is molded for the last flexible tube substrate, is taken out of the continuous molding machine. Thereafter, the joint member is removed from the flexible tube substrates 1, and then the flexible tube substrates 1 are separated from each other one by one. Subsequently, for each of the separated flexible tube substrates 1, a top coat layer is coated onto the resin layer 15, thereby completing the flexible tube 3a. The completed flexible tube 3a is transported to undergo a step of being assembled into an electronic endoscope.

[Top coat layer]

For the flexible tube 3a of the present embodiment, the top coat layer 16 is used. As the resin of the top coat layer 16, a polymer compound I derived from monomers including the following A to C is used.

| | |
|---|---|
| [A: hydroxyl group-free acrylate having a molecular weight equal to or greater than 110 | 100 parts by mass] |
| [B: hydroxyl group-free acrylate having a molecular weight equal to or greater than 80 and equal to or less than 110 | 10 parts by mass to 200 parts by mass] |
| [C: hydroxyalkyl acrylate | 1 part by mass to 60 parts by mass] |

The molecular weight of the acrylate (A) is equal to or greater than 110, preferably equal to or greater than 115, and more preferably equal to or greater than 120. The upper limit thereof is not particularly limited, but it is preferably equal to or less than 150, and more preferably equal to or less than 140.

The molecular weight of the acrylate (B) is equal to or greater than 80, and preferably equal to or greater than 85. The upper limit thereof is equal to or less than 110, and preferably equal to or less than 105.

The amount of the acrylate (B) used is equal to or greater than 10 parts by mass, preferably equal to or greater than 20 parts by mass, more preferably equal to or greater than 40 parts by mass, even more preferably equal to or greater than 60 parts by mass, still more preferably equal to or greater than 80 parts by mass, and yet more preferably equal to or greater than 100 parts by mass, with respect to the acrylate (A). The upper limit thereof is equal to or less than 200 parts by mass, preferably equal to or less than 170 parts by mass, more preferably equal to or less than 160 parts by mass, and particularly preferably equal to or less than 150 parts by mass.

The amount of the acrylate (C) used is equal to or greater than 1 part by mass, preferably equal to or greater than 5 parts by mass, and even more preferably equal to or greater than 7 parts by mass, with respect to the acrylate (A). The upper limit is equal to or less than 60 parts by mass, preferably equal to or less than 50 parts by mass, more preferably equal to or less than 40 parts by mass, even more preferably equal to or less than 30 parts by mass, and particularly preferably equal to or less than 20 parts by mass.

Herein, the mixing ratio can be evaluated as a copolymerization ratio of the polymer compound.

The monomer A is preferably butyl acrylate, butyl methacrylate, pentyl acrylate, pentyl methacrylate, hexyl acrylate, or hexyl methacrylate, and more preferably butyl acrylate or hexyl acrylate.

The monomer B is preferably ethyl acrylate, methyl acrylate, or methyl methacrylate.

The monomer C is preferably hydroxyethyl acrylate, hydroxyethyl methacrylate, hydroxymethyl acrylate, hydroxymethyl methacrylate, hydroxypropyl acrylate, hydroxypropyl methacrylate, hydroxybutyl acrylate, or hydroxybutyl methacrylate, and more preferably hydroxyethyl acrylate, hydroxymethyl acrylate, hydroxypropyl acrylate, or hydroxybutyl acrylate.

The polymer compound I may be either a polymer containing only the monomers A to C or a polymer containing other monomers in addition to the monomers A to C. As the other monomers, it is possible to appropriately use monomers that can be incorporated into this type of resin. Examples of the other monomers include acrylic acid monomers and methacrylic acid monomers. In addition, styrene or various vinyl monomers may be used.

The top coat layer 16 may be either a resin layer containing only the polymer compound I or a layer containing other components as described below. The content of the polymer compound I in the solid content in the resin composition for the top coat layer 16 is preferably equal to or greater than 10% by mass, and more preferably equal to or greater than 20% by mass. The upper limit thereof is preferably equal to or less than 80% by mass.

In the polymer compound I, the content of hydroxyl groups expressed in terms of a hydroxyl value is preferably equal to or greater than 30 mg KOH/g, and more preferably equal to or greater than 60 mg KOH/g. The upper limit thereof is preferably within a range equal to or less than 120 mg KOH/g, and more preferably equal to or less than 100 mg KOH/g. Upon a condition where the hydroxyl value is within the above range, it is preferable since sufficient crosslinking density is obtained, and the hardness of the film does not become too high.

The weight average molecular weight of the polymer compound I is preferably equal to or greater than 10,000, more preferably equal to or greater than 100,000, and particularly preferably equal to or greater than 200,000. The upper limit thereof is preferably equal to or less than 2,000,000. The molecular weight within the above range is preferable since the film does not become too hard and does not become brittle, and coating properties become excellent. Unless otherwise specified, the molecular weight of the polymer compound used for the top coat layer 16 is set according to the condition defined in the section of the resin layer which will be described later. As an eluent, tetrahydrofuran is used.

For the top coat layer 16 according to the present embodiment, it is preferable to concurrently use the polymer compound I and a curing agent, and a cured film composed of these is preferably used as the top coat layer 16. As the curing agent, an isocyanate compound is preferably used. The isocyanate compound is preferably a hexamethylene diisocyanate derivative having active isocyanate on the terminal thereof and having functional groups in a number equal to or greater than 2.5 (preferably equal to or less than 3.5). The number of functional groups of isocyanate within the above range is preferable since a strong and flexible film can be manufactured.

The hexamethylene diisocyanate derivative is not particularly limited, but is preferably trimerized hexamethylene diisocyanate (hereinafter, referred to as "trimerized HDI"), a compound obtained by partially adding trimethylolporpane to hexamethylene diisocyanate (hereinafter, referred to as "TMP-HDI"), a compound having an active isocyanate group on the terminal thereof that is obtained by partially adding an active hydrogen group-containing compound to hexamethylene diisocyanate (hereinafter, referred to as a "prepolymer"). Furthermore, these organic isocyanates can be mixed with blocked polyisocyanate in which an active portion is blocked by known blocking agents such as alcohols, phenols, and amines.

The amount of the curing agent added to and mixed with the polymer compound I is set such that a ratio of the equivalent of the reacting curing agent to the equivalent of the reacting main agent (NCO/OH) is preferably within a range equal to or greater than 0.8, and more preferably equal to or greater than 0.95. The upper limit thereof is preferably equal to or less than 1.5, and more preferably equal to or less than 1.3. With the ratio within the above range, a reaction sufficiently occurs, and thereby a strong film is obtained. Moreover, the ratio within the above range is preferable since unreacted functional groups do not remain in the cured film. Provided that the amount of the polymer compound used in the top coat layer 16 is 100 parts by mass, the content of the curing agent is preferably equal to or greater than 0.01 parts by mass, and more preferably equal to or greater than 1 part by mass, in terms of mass ratio. The upper limit thereof is preferably equal to or less than 50 parts by mass.

The resin composition for the top coat layer 16 is coated onto the aforementioned molding resin composition (elastomer layer). Accordingly, in view of facilitating the coating operation, it is preferable to use a solvent, although this is not an essential requirement. Various solvents can be used, and for example, aromatic hydrocarbons such as xylene, ketones such as methyl isobutyl ketone, are preferable. It is desirable to select these organic solvents in consideration of the overall state of a substance to be coated, the evaporation speed, the operation environment.

Furthermore, for preparing the composition of the present invention, various apparatuses generally used for coating and making a film may be used. At this time, if necessary, a catalyst, a dispersion stabilizer, a viscosity-adjusting agent, an ultraviolet stabilizer, a moisture absorbent, can be added. In addition, these additives can be premixed with the components of the main agent.

The curable resin composition for the top coat layer 16 that is obtained as described above is coated onto the resin layer 15 by a method using a spray, a brush, or a roller or by a method such as dipping, and in this way, a film having a predetermined thickness can be formed. After coating, the resultant is left at room temperature for a predetermined amount of time, thereby forming the top coat layer 16. Moreover, the top coat layer 16 may be cured by being heated at a temperature equal to or less than 200°C (preferably equal to or less than 120°C). Heating the top coat layer 16 at the temperature within the above range is preferable since a secondary crosslinking reaction of isocyanate does not occur.

The top coat layer 16 of the present embodiment is preferable since it has a high elastic modulus and a smooth surface and has excellent chemical resistance. A storage modulus $E_T$' of only the top coat layer 16 (material forming the top coat layer 16) is desirably smaller than a storage modulus $E_R$' of the material forming the resin layer 15 (outermost layer when the resin layer 15 includes a plurality of layers). Specifically, $E_T$' is preferably equal to or less than 20 MPa, more preferably equal to or less than 10 MPa, and particularly preferably equal to or less than 5 MPa. Keeping the elastic modulus of the top coat layer 16 low is preferable since it is possible to take advantage of characteristics of the resin layer 15. Herein, the storage modulus is measured based on JIS-K7244-4 similarly to the resin layer 15 which will be described later.

The lower limit of the storage modulus $E_T$' is not particularly limited, but is preferably equal to or greater than 0.1 MPa, more preferably equal to or greater than 0.5 MPa, and particularly preferably equal to or greater than 1 MPa.

$E_R$'-$E_T$', which shows a difference between the storage modulus $E_T$' of the material of the top coat layer 16 and the storage modulus $E_R$' of the material of the resin layer 15 which will be described later, is equal to or greater than 1 MPa, more preferably equal to or greater than 10 MPa, even more preferably equal to or greater than 80 MPa, and particularly preferably equal to or greater than 100 MPa. The upper limit of the difference is not particularly limited, but is practically equal to or less than 200 MPa.

When the resin layer 15 is divided into a plurality of layers, the storage modulus ER' of the resin layer 15 refers to E' of the resin layer 15 immediately below the top coat layer 16. For example, in the case where a polyester elastomer layer is positioned immediately below the top coat layer 16, E' of the polyester elastomer serves as $E_R$' even if a polyurethane elastomer layer is placed below (inner side) the polyester elastomer layer.

From the viewpoint of improving the performance of the resin layer 15, it is preferable for the top coat layer 16 to have a small thickness. The thickness is preferably equal to or less than 200 $\mu$m, more preferably equal to or less than 100 $\mu$m, and particularly preferably equal to or less than 50 $\mu$m. The lower limit thereof is not particularly limited, but is practically equal to or greater than 10 $\mu$m.

In the present specification, regarding options of substituents or linking groups of compounds as well as each of the technological matters such as temperature and thickness, even though the list thereof is described independently, they can be combined with each other.

The top coat layer 16 according to the present embodiment with the configuration described as above provides excellent performance in the flexible tube 3a. Particularly, it can realize excellent resilience in cooperation with the characteristics of the resin layer 15 of the inside thereof. The reason is assumed to be as below. When bending resilience of a resin is measured, the repulsive force gradually decreases with the passage of time compared to the initial repulsive force (force at an initial stage). Accordingly, when an endoscope is operated and inserted into the human body, a force that an operator applies to operate the endoscope is not transmitted well to the soft portion in some cases. In most cases, the decrease in the repulsive force results from the physical properties of the resin, and the smaller the decrease, the easier it becomes for the applied force to be transmitted. However, a resin, in which the decrease in the repulsive force is small, is likely to have poor flexibility. If such a resin is used, a great strain is imposed on the human body.

The top coat layer 16 tends to be made into a thick layer such that it secures resistance to a disinfectant solution having powerful disinfectant activity. However, when the top coat resin is hard, thick, and poor in transmission of force (decrease in force is great), even if a resin having excellent performance is selected for the resin layer 15, the excellent performance becomes useless in some cases.

However, according to the present embodiment the top coat layer 16 can obtain sufficient chemical resistance or abrasion resistance even though the thickness thereof is not excessively increased. Furthermore, the top coat layer 16 can realize excellent resilience by preferably utilizing the performance of the molding resin layer 15 inside thereof. Particularly, the top coat layer 16 combined with the resin layer 15 containing a resin having a high degree of resilience such as a polyester elastomer is preferable since it is possible to take advantage of the characteristics of the resin layer 15 without impairing the characteristics. Such an effect is particularly preferable for the flexible tube 3a constituted by the resin layer 15, which consists of two layers with a gradient, and a flexible tip. In this case, it is preferable to use a polyester elastomer layer as the outer layer 18 and to dispose the top coat layer 16 according to the present embodiment on the outer layer 18 (surface of the outer side).

Furthermore, the surface of the top coat layer 16 according to the present embodiment has excellent sliding properties, and accordingly, strain imposed on the human body is reduced.

(Resin layer)

In the flexible tube 3a of the present embodiment, the resin layer 15 (referred to as a "molding resin layer" in some cases so as to be differentiated from the top coat layer 16) may consist of a single layer or a plurality of layers.

Hereinafter, an embodiment will be described in which the resin layer 15 consists of a plurality of specific layers including a layer A and a layer B.

The content of a polyester elastomer in the resin component constituting the layer A (preferably an outermost layer) of the resin layer 15 is preferably equal to or greater than 50% by mass, more preferably equal to or greater than 55% by mass, even more preferably equal to or greater than 60% by mass, and still more preferably equal to or greater than 65% by mass. The content of the polyester elastomer of the layer A may be 100% by mass. However, the content is preferably equal to or less than 90% by mass, more preferably equal to or less than 80% by mass, and even more preferably equal to or less than 75% by mass. Under a condition in which the content is within the aforementioned preferable range, and a soft resin is blended as the balance, better flexibility can be achieved.

The layer A may contain at least one kind of softer polyurethane elastomer and polyamide elastomer. Particularly, the layer A preferably contains at least a polyurethane elastomer. The content of the polyurethane elastomer in the resin component of the layer A of the resin layer 15 is preferably equal to or greater than 5% by mass, more preferably equal to or greater than 10% by mass, even more preferably equal to or greater than 15% by mass, still more preferably equal to or greater than 20% by mass, and yet more preferably equal to or greater than 25% by mass. Furthermore, the content of the polyurethane elastomer in the resin component of the layer A of the resin layer 15 is preferably equal to or less than 50% by mass, more preferably equal to or less than 40% by mass, and even more preferably equal to or less than 35% by mass.

Considering weather resistance or oxidation resistance, the layer A may contain a hindered phenol compound or a hindered amine compound. The content of such a compound is not particularly limited. However, the content of the hindered phenol compound is preferably equal to or greater than 0.01 parts by mass, and more preferably equal to or greater than 0.1 parts by mass, with respect to 100 parts by mass of the resin component in the layer A. The upper limit thereof is preferably equal to or less than 7 parts by mass, and more preferably equal to or less than 5 parts by mass. The content of the hindered amine compound is preferably equal to or greater than 0.01 parts by mass, and more preferably equal to or greater than 0.1 parts by mass, with respect to 100 parts by mass of the resin component. The upper limit thereof is preferably equal to or less than 7 parts by mass, and more preferably equal to or less than 5 parts by mass.

When the resin layer 15 consists of a plurality of layers, at least one layer (hereinafter, this layer will be referred to as a "layer B") other than the layer A (preferably an outermost layer) preferably contains a polyurethane elastomer or a polyamide elastomer. The layer B more preferably contains at least a polyurethane elastomer. The layer B preferably contains a polyurethane elastomer as a main component, and in this case, the content of the polyurethane elastomer in the resin component of the layer B is preferably equal to or greater than 50% by mass, more preferably equal to or greater than 70% by mass, even more preferably equal to or greater than 80% by mass, and still more preferably equal to or greater than 90% by mass. It is also preferable for the resin component in the layer B to be composed of only the polyurethane elastomer. However, in the case where the layer B is composed of the polyurethane elastomer and other material, the other material is preferably constituted by a polyamide elastomer and/or a urethane elastomer.

-Physical properties-

The molecular weight of the used elastomer is not particularly limited. However, from the viewpoint of forming a preferable hard segment and causing the hard segment to excellently interact with a soft segment formed by a chain extender, the molecular weight is preferably 10,000 to 1,000,000, more preferably 20,000 to 500,000, and particularly preferably 30,000 to 300,000.

In the present embodiment, unless otherwise specified, the molecular weight of an elastomer means a weight average molecular weight. The weight average molecular weight can be measured as a molecular weight expressed in terms of polystyrene by means of GPC. At this time, HLC-8220 (manufactured by TOSOH CORPORATION) is used as a GPC apparatus; chloroform is used as an eluent in the case of the urethane elatomer; NMP (N-methyl-2-pyrrolidone) is used as an eluent in the case of the polyurethane elastomer; m-cresol/chloroform (manufactured by Shonan Wako Pure Chemical Corporation) is used as an eluent in the case of the polyamide elastomer; G3000HXL + G2000HXL are used as columns; and the weight average molecular weight is detected by RI at 23°C at a flow rate of 1 mL/min.

It is preferable to appropriately set physical properties of the layer B (preferably an inner layer). For example, A hardness thereof measured based on JIS-K7215 is preferably equal to or greater than 40, more preferably equal to or greater than 50, and particularly preferably equal to or greater than 60. The range of the upper limit thereof is preferably equal to or less than 98, more preferably equal to or less than 95, and particularly preferably equal to or less than 90.

A storage modulus $E_B'$ of the layer B is preferably equal to or greater than 1 MPa, more preferably equal to or greater than 2 MPa, and particularly preferably equal to or greater than 3 MPa. The range of the upper limit thereof is preferably equal to or less than 150 MPa, more preferably equal to or less than 100 MPa, and particularly preferably equal to or less than 50 MPa. A loss modulus $E_B''$ of the layer B is preferably equal to or greater than 0.1 MPa, more preferably equal to or greater than 0.3 MPa, and particularly preferably equal to or greater than 0.5 MPa. The range of the upper limit thereof is preferably equal to or less than 20 MPa, more preferably equal to or less than 10 MPa, and particularly preferably equal to or less than 5 MPa. A loss tangent of the layer B is preferably equal to or greater than 0.01, more preferably equal to or greater than 0.03, and particularly preferably equal to or greater than 0.05. The range of the upper limit thereof is preferably equal to or less than 1, more preferably equal to or less than 0.5, and particularly preferably equal to or less than 0.3. Herein, in the present specification, unless otherwise specified, the value relating to viscoelasticity is a value at 25°C and measured by the method based on JIS-K7244-4.

It is preferable to appropriately set physical properties of the layer A (preferably an outer layer) of the resin layer 15. For example, D hardness thereof measured based on JIS-K7215 is preferably equal to or greater than 20, more preferably equal to or greater than 25, and particularly preferably equal to or greater than 30. The range of the upper limit thereof is preferably equal to or less than 80, more preferably equal to or less than 70, and particularly preferably equal to or less than 60.

A storage modulus $E_A'$ of the layer A of the resin layer 15 is preferably equal to or greater than 1 MPa, more preferably equal to or greater than 5 MPa, and particularly preferably equal to or greater than 10 MPa. The range of the upper limit thereof is preferably equal to or less than 1 GPa, more preferably equal to or less than 500 MPa, and particularly preferably equal to or less than 300 MPa. A loss modulus $E_A''$ of the layer A of the resin layer is preferably equal to or greater than 0.1 MPa, more preferably equal to or greater than 0.5 MPa, and particularly preferably equal to or greater than 1 MPa. The range of the upper limit thereof is preferably equal to or less than 100 MPa, more preferably equal to or less than 50 MPa, and particularly preferably equal to or less than 30 MPa. A loss tangent of the layer A of the resin layer 15 is preferably equal to or greater than 0.01, more preferably equal to or greater than 0.03, and particularly preferably equal to or greater than 0.05. The range of the upper limit thereof is preferably equal to or less than 1, more preferably equal to or less than 0.5, and particularly preferably equal to or less than 0.3.

A difference obtained by subtracting the storage modulus $E_B'$ of the inner layer (layer B) from the storage modulus $E_A'$ of the outer layer (layer A) is preferably greater than 0 MPa, more preferably equal to or greater than 20 MPa, even more preferably equal to or greater than 30 MPa, and particularly preferably equal to or greater than 50 MPa. The upper limit of the difference is preferably equal to or less than 400 MPa, more preferably equal to or less than 200 MPa, and particularly preferably equal to or less than 150 MPa.

A value of 100% modulus of the layer B is preferably equal to or greater than 0.5 MPa, and more preferably equal to or greater than 1 MPa, and particularly preferably equal to or greater than 1.5 MPa. The range of the upper limit thereof is preferably equal to or less than 20 MPa, more preferably equal to or less than 15 MPa, and particularly preferably equal to or less than 10 MPa.

A value of 100% modulus of the layer A of the resin layer 15 is preferably equal to or greater than 1 MPa, more preferably equal to or greater than 1.5 MPa, and particularly preferably equal to or greater than 2 MPa. The range of the upper limit thereof is preferably equal to or less than 30 MPa, more preferably equal to or less than 25 MPa, and particularly preferably equal to or less than 20 MPa.

In the present specification, unless otherwise specified, the value of modulus is a value at 25°C and measured by the method based on JIS-K7311.

The resin layer 15 may or may not be crosslinked. However, it is preferable for the elastomer constituting the resin layer 15 not to be substantially crosslinked. Herein, "not to be substantially crosslinked" means that the elastomer is not crosslinked, and the resin does not have a branched structure within a detectable range of NMR. If the elastomer of the resin layer 15 (particularly, the second layer (outer layer)) according to the present embodiment is not substantially crosslinked, it is preferable since the elastomer provides the performances such as flexibility and bending durability, as the resin layer of the flexible tube for an endoscopic medical apparatus.

Considering the relationship between the resin layer 15 and the top coat layer 16 adopted in the present embodiment, the resin layer 15 preferably has a small thickness. The thickness is preferably equal to or less than 1,000 μm, more preferably equal to or less than 500 μm, and particularly preferably equal to or less than 400 μm. The lower limit thereof is not particularly limited, but is practically a thickness equal to or greater than 200 μm.

The flexible tube 3a according to the present embodiment can be used not only for an endoscope but also for an endoscopic medical apparatus in various ways. For example, the flexible tube 3a can be used for an apparatus in which a clip or a wire is mounted on the tip of an endoscope or for an instrument equipped with a basket or a brush, and at this time, the flexible tube 3a exerts its excellent effect. Herein, the endoscopic medical apparatus includes not only the aforementioned medical apparatus that has an endoscope as a basic structure but also a medical apparatus and a diagnostic apparatus, such as remote-controlled medical apparatuses, that have flexibility and are used by being introduced into body cavities.

[Examples]

Hereinafter, the present invention will be more specifically described based on examples, but the present invention is not limited to the examples. Herein, unless otherwise specified, "part(s)" and "%" listed without a unit is based on mass. Furthermore, unless otherwise specified, the following tests are performed in an environment of 25°C (room temperature) and 50% RH.

(Example 1/Comparative Example 1)

By mixing the following components at a ratio (part(s) by mass) described in the following formulations 1 and 2, resin mixtures (for an outer layer and an inner layer respectively) were prepared. Each of the resin mixtures was subjected to a melting/kneading process by using a twin-screw kneader (trade name: KZW15-30MG) manufactured by TECHNOVEL CORPORATION at a barrel temperature set to 210°C and a screw rotation frequency of 100 rpm. The ejected molten resin strand was cooled in a water tank and then made into pellet-shaped samples by using a pelletizer.

| Formulation 1···molding resin 1 for outer layer (storage modulus: about 100 MPa) | |
| --- | --- |
| PE1 | 70 parts by mass |
| PU1 | 30 parts by mass |
| HP-1 | 5 parts by mass |
| HA-1 | 0.5 parts by mass |

| Formulation 2···molding resin 2 for inner layer (storage modulus: about 10 MPa) | |
| --- | --- |
| PU2 | 100 parts by mass |
| HP-1 | 0.5 parts by mass |
| HA-1 | 0.5 parts by mass |

PE1: Hytrel 4047 (40D) manufactured by DU PONT-TORAY CO., LTD (weight average molecular weight: 123,000, 100% modulus: 25 MPa) Polyurethane elastomer (number in the bracket indicates D hardness: JIS-K7215)
PU1: Pandex T-2190 (92 A) manufactured by DIC Bayer Polymer Ltd. (weight average molecular weight: 189,000, 100% modulus: 11MPa)
PU2: Pandex T-5865 (65A) manufactured by DIC Bayer Polymer Ltd. (weight average molecular weight: 172,000, 100% modulus: 2.3 MPa)
HP-1: IRG 1330: Irganox 1330 (manufactured by BASF Corporation)
HA-1: Tinuvin 770DF (manufactured by BASF Corporation)

The aforementioned resin was introduced in a continuous molding machine, thereby preparing a flexible tube for endoscope. Specifically, a flexible tube substrate having a diameter of 12.0 mm and a length of 120 cm was covered with the resin mixture (composition) for inner layer of the formulation 2 and the resin mixture (composition) for outer layer of the formulation 1 in this order. The thickness of the resin layer was 0.4 mm. At this time, the thickness ratio between the inner layer and the outer layer at the tip and rear end was set as below.

| | Tip | Rear end | |
| --- | --- | --- | --- |
| Inner layer | 80 | 20 | Formulation 2 (PU) |
| Outer layer | 20 | 80 | Formulation 1 (PE/PU) |

A polymerization reaction was caused by mixing monomers together in the amount described in Table 1, thereby obtaining respective polymer compounds for a top coat layer. 100 parts by mass of each of the polymer compounds, 41.08 parts by mass of MTP-HDI as a curing agent component, and 0.0025 parts by mass of a urethanization catalyst were dissolved together in 100 parts by mass of a solvent containing a mixture of xylene and methyl isobutyl ketone (mixing ratio: 1:1), thereby preparing a composition for top coat layer. This curable resin composition was uniformly coated onto the resin layer on the flexible tube substrate prepared as above, such that the thickness after curing became 0.03 mm. In this state, the resultant was dried and cured by being heated at 60°C for 24 hours, thereby forming a top coat.

[Chemical resistance]

A sample was prepared by coating the surface of the molding resin layer with a coating liquid by means of dip coating, and the sample was cut in size of 1 cm × 10 cm, thereby preparing specimens. The specimens were dipped into a 0.3% aqueous peracetic acid solution at 50°C for 150 hours, and then the surface thereof was thoroughly washed with water. Thereafter, the specimens were dried for 24 hours under a condition of 23°C × 50% RH and then subjected to a tensile test with an elongation rate of 50% by using Tensilon (Fig. 4).

AA: The specimen was not ruptured even in a tensile test with an elongation rate of 150%.
A: The specimen was not ruptured in a tensile test with an elongation rate of 100%.
B: The specimen was not ruptured in a tensile test with an elongation rate of 50%.
C: The specimen was ruptured in a tensile test with an elongation rate of 50%.

[Sliding properties]

Sliding properties were measured by using the sliding tester of Fig. 3. Specifically, a sample sheet which was obtained by coating a coating material onto a sheet and curing the resultant was used. The sample size was 200 mm × 100 mm × 0.5 mm (HWD), and the measurement was performed at room temperature (25°C). A weight made of aluminum was put on the sheet, and in this state, the sheet was slowly tilted, and an angle θ at a point in time when the weight started to move was measured. From the measured angle θ, a coefficient of static friction was calculated.

A: θ is less than 60°.
B: θ is equal to or greater than 60° and less than 90°
C: θ is equal to or greater than 90°.

[Resilience]

A position 50 cm away from the tip portion and a position 70 cm away from the tip portion were fixed. In this state, the flexible tube was pushed by 15 mm at a position (central portion of the flexible tube) 60 cm away from the tip portion. In this way, a ratio of a repulsive force (B) after 30 seconds to a repulsive force (A) after 0.1 seconds was measured as the resilience (%).

$$[\text{Resilience (\%)}] = (B) / (A) \times 100$$

A: The resilience is equal to or greater than 80%.
B: The resilience is equal to or greater than 70% and less than 80%.
C: The resilience is less than 70%.

[Abrasion resistance]

A coating liquid was coated onto the molding resin layer by means of dip coating, thereby obtaining a sample. By using a reciprocating abrasion tester (model name: TYPE 30S) manufactured by HEIDON, the sample was reciprocated 40,000 times under a load of 200 g, and the state of the samples after the test was observed. The sample size was 100 mm × 50 mm × 0.5 mm (HWD), and the test was performed at room temperature (25°C).

A: The coat was not cracked or peeled.
B: The coat was cracked or peeled, but the underlying layer was not seen.
C: The coat was cracked or peeled, and the underlying layer was seen.

[Elasticity test]

As a tester, the same Tensilon apparatus (Fig. 4) as used in the chemical resistance test was used. Moreover, the shape and size of the sample were the same as those of the sample used in the chemical resistant test. Regarding 20% modulus, the sample was clamped between chucks in the Tensilon apparatus and elongated 20%, and stress data obtained at this time was taken. The test was performed at room temperature (25°C).
The lower the stress caused in a sample, the easier it becomes for the sample to be elongated with a weak force, and the better the flexibility.

A: The tensile stress is less than 3 MPa.
B: The tensile stress is equal to or greater than 3 MPa and less than 10 MPa.
C: The tensile stress is equal to or greater than 10 MPa.

[Storage modulus (E')]

The storage modulus was measured based on JIS-K7244-4. Specifically, the same sheet sample as shown in Fig. 3 was clamped between chucks at both sides so as to measure the storage modulus. The storage modulus was measured by using an apparatus with a model name of DVA-225 manufactured by IT Instrumentation & Control Company at a measurement frequency of 1 Hz.

[Table 1]

| No. | Monomer [part by mass | | | | | Test | | | | | |
|---|---|---|---|---|---|---|---|---|---|---|---|
| | (A) | (B-1) | (B-2) | (C) | Other | Chemical resistance | Abrasion resistance | Resilience | Sliding properties | 200% Mod | E' |
| 101 | BA [100] | EA [100] | MMA [35] | HEMA [10] | | AA | A | A | A | A | 4 |
| 102 | BA [100] | | MMA [35] | HEMA [10] | | AA | A | A | A | B | 10 |
| C01 | iBMA [100] | | | HEMA [10] | MA [10] | AA | C | A | A | B | 1 |
| C02 | BA [100] | EA [100] | | | | AA | C | A | A | A | 1 |
| C03 | | | MMA [100] | HEMA [100] | | AA | A | C | A | C | 104 |

EP 2 896 635 B1

14

<Note for table>

Test No.: A test number beginning with C indicates a comparative example.
BA: n-butyl acrylate
EA: ethyl acrylate
MA: methyl acrylate
MMA: methyl methacrylate
HEMA: hydroxyethyl acrylate
iBMA: isobutyl methacrylate

As is evident from the above results, the flexible tube of the present invention realizes a high degree of chemical resistance and sliding properties and has excellent flexibility. Accordingly, the flexible tube has excellent durability. Furthermore, when a medical apparatus using the flexible tube is used for making a diagnosis in a body cavity, excellent operability is provided to a physician, and the strain that a patient suffers from can be reduced.

A top coat layer was formed in the same manner as described above, except that hexyl acrylate was used instead of the n-butyl acrylate, and methyl acrylate was used instead of ethyl acrylate. As a result of performing tests regarding the same items as described above (chemical resistance, sliding properties, resilience, and abrasion resistance), excellent performance was obtained.

**Claims**

1. A flexible tube comprising:

    a flexible tube substrate that has a cylindrical shape and flexibility;
    a resin layer that covers the flexible tube substrate; and
    a top coat layer that covers the resin layer,
    wherein the top coat layer contains a polymer compound I derived from monomers including the following A to C,
    Monomer A: hydroxyl group-free acrylate having a molecular weight equal to or greater than 110 100 parts by mass,
    Monomer B: hydroxyl group-free acrylate having a molecular weight equal to or greater than 80 and less than 110 10 parts by mass to 200 parts by mass,
    Monomer C: hydroxyalkyl acrylate 1 part by mass to 60 parts by mass.

2. The flexible tube according to claim 1,
    wherein the monomer A is butyl acrylate or hexyl acrylate.

3. The flexible tube according to claim 1 or 2,
    wherein the monomer B is ethyl acrylate, methyl acrylate, or methyl methacrylate.

4. The flexible tube according to any one of claims 1 to 3,
    wherein the monomer C is hydroxyethyl acrylate, hydroxymethyl acrylate, hydroxypropyl acrylate, or hydroxybutyl acrylate.

5. The flexible tube according to any one of claims 1 to 4,
    wherein the resin layer contains at least one kind selected from among a urethane elastomer, a polyurethane elastomer, and a polyamide elastomer.

6. The flexible tube according to any one of claims 1 to 5,
    wherein the top coat layer is a layer formed by curing the polymer compound I with a curing agent.

7. The flexible tube according to any one of claims 1 to 6,
    wherein a storage modulus $E_T'$ of the material of the top coat layer is smaller than a storage modulus $E_R'$ of the material of the resin layer, wherein the storage modulus is measured at 25°C by the method based on JIS-K7244-4.

8. The flexible tube according to claim 7,

wherein the storage modulus $E_T'$ of the material of the top coat layer is equal to or greater than 1 MPa and equal to or less than 20 MPa.

9. The flexible tube according to claim 7 or 8,
wherein the storage modulus $E_R'$ of the material of the resin layer is equal to or greater than 1 MPa and equal to or less than 500 MPa.

10. The flexible tube according to claim 7 or 8,
wherein $E_R'$ - $E_T'$, which represents a difference between the storage modulus $E_T'$ of the material of the top coat layer and the storage modulus $E_R'$ of the material of the resin layer, is equal to or greater than 1 MPa and equal to or less than 200 MPa.

11. The flexible tube according to any one of claims 1 to 10,
wherein a thickness of the top coat layer is equal to or greater than 10 $\mu$m and equal to or less than 200 $\mu$m.

12. The flexible tube according to any one of claims 1 to 11,
wherein the resin layer has an inner layer and an outer layer, and a thickness ratio between the inner layer and the outer layer changes with a gradient in an axial direction of the flexible tube substrate.

13. The flexible tube according to any one of claims 1 to 12,
wherein the resin layer has an inner layer and an outer layer, a storage modulus of the inner layer is equal to or greater than 1 MPa and equal to or less than 150 MPa, a storage modulus of the outer layer is equal to or greater than 1 MPa and equal to or less than 500 MPa, and a difference obtained by subtracting the storage modulus of the inner layer from the storage modulus of the outer layer is equal to or greater than 20 MPa and equal to or less than 200 MPa.

14. The flexible tube according to claim 12,
wherein in a first end portion of the flexible tube substrate, the thickness ratio between the inner layer and the outer layer is 5:95 to 40:60 (inner layer:outer layer), in a second end portion of the flexible tube substrate, the thickness ratio is 95:5 to 60:40 (inner layer:outer layer), and inversion of the thickness ratio occurs between the first end portion and the second end portion.

15. The flexible tube according to any one of claims 1 to 14, wherein the flexible tube is configured to be used for an endoscopic medical apparatus.

16. An endoscopic medical apparatus comprising the flexible tube according to any one of claims 1 to 15.


**Patentansprüche**

1. Flexibler Schlauch aufweisend:

   ein flexibles Schlauchträgermaterial, das eine zylindrische Gestalt und Flexibilität besitzt;
   eine Harzschicht, die das flexible Schlauchträgermaterial bedeckt; und
   eine Deckschicht, die die Harzschicht bedeckt,
   wobei die Deckschicht eine Polymerverbindung I auf der Basis von Monomeren, zu denen die folgenden A bis C gehören, enthält,
   Monomer A: Hydroxylgruppen-freies Acrylat mit einem Molekulargewicht von 110 oder größer 100 Masseteile,
   Monomer B: Hydroxylgruppen-freies Acrylat mit einem Molekulargewicht von 80 oder größer und geringer als 110 10 Masseteile bis 200 Masseteile,
   Monomer C: Hydroxyalkyl-acrylat 1 Masseteil bis 60 Masseteile.

2. Flexibler Schlauch nach Anspruch 1,
   wobei das Monomer A Butyl-acrylat oder Hexyl-acrylat ist.

3. Flexibler Schlauch nach Anspruch 1 oder 2,
   wobei das Monomer B Ethyl-acrylat, Methyl-acrylat oder Methyl-methacrylat ist.

**4.** Flexibler Schlauch nach einem der Ansprüche 1 bis 3,
wobei das Monomer C Hydroxyethyl-acrylat, Hydroxymethyl-acrylat, Hydroxypropyl-acrylat oder Hydroxybutyl-acrylat ist.

**5.** Flexibler Schlauch nach einem der Ansprüche 1 bis 4,
wobei die Harzschicht mindestens eine Art enthält, die ausgewählt ist aus einem Urethan-Elastomer, einem Polyurethan-Elastomer und einem Polyamid-Elastomer.

**6.** Flexibler Schlauch nach einem der Ansprüche 1 bis 5,
wobei die Deckschicht eine durch Vernetzen der Polymerverbindung I mit einem Vernetzungsmittel ausgebildete Schicht ist.

**7.** Flexibler Schlauch nach einem der Ansprüche 1 bis 6,
wobei ein Speichermodul $E_T'$ des Materials der Deckschicht kleiner ist als ein Speichermodul $E_R'$ des Materials der Harzschicht, wobei der Speichermodul bei 25°C nach dem auf JIS-K7244-4 basierenden Verfahren gemessen wird.

**8.** Flexibler Schlauch nach Anspruch 7,
wobei der Speichermodul $E_T'$ des Materials der Deckschicht gleich 1 MPa oder größer und gleich 20 MPa oder kleiner ist.

**9.** Flexibler Schlauch nach Anspruch 7 oder 8,
wobei der Speichermodul $E_R'$ des Materials der Harzschicht gleich 1 MPa oder größer und gleich 500 MPa oder kleiner ist.

**10.** Flexibler Schlauch nach Anspruch 7 oder 8,
wobei $E_R'$ - $E_T'$, was eine Differenz zwischen dem Speichermodul $E_T'$ des Materials der Deckschicht und dem Speichermodul $E_R'$ des Materials der Harzschicht repräsentiert, gleich 1 MPa oder größer und gleich 200 MPa oder kleiner ist.

**11.** Flexibler Schlauch nach einem der Ansprüche 1 bis 10,
wobei eine Dicke der Deckschicht gleich 10 $\mu$m oder größer und gleich 200 $\mu$m oder kleiner ist.

**12.** Flexibler Schlauch nach einem der Ansprüche 1 bis 11,
wobei die Harzschicht eine innere Schicht und eine äußere Schicht hat, und ein Dickenverhältnis zwischen der inneren Schicht und der äußeren Schicht sich in axialer Richtung des flexiblen Schlauchträgermaterials mit einem Gradienten verändert.

**13.** Flexibler Schlauch nach einem der Ansprüche 1 bis 12,
wobei die Harzschicht eine innere Schicht und eine äußere Schicht hat, ein Speichermodul der inneren Schicht gleich 1 MPa oder größer und gleich 150 MPa oder kleiner ist, ein Speichermodul der äußeren Schicht gleich 1 MPa oder größer und gleich 500 MPa oder kleiner ist, und eine durch Subtrahieren des Speichermoduls der inneren Schicht von dem Speichermodul der äußeren Schicht erhaltene Differenz gleich 20 MPa oder größer und gleich 200 MPa oder kleiner ist.

**14.** Flexibler Schlauch nach Anspruch 12,
wobei in einem ersten Endbereich des flexiblen Schlauchträgermaterials das Dickenverhältnis zwischen der inneren Schicht und der äußeren Schicht 5:95 bis 40:60 (innere Schicht:äußere Schicht) beträgt, in einem zweiten Endbereich des flexiblen Schlauchträgermaterials das Dickenverhältnis 95:5 bis 60:40 (innere Schicht:äußere Schicht) beträgt, und zwischen dem ersten Endbereich und dem zweiten Endbereich eine Umkehrung des Dickenverhältnisses auftritt.

**15.** Flexibler Schlauch nach einem der Ansprüche 1 bis 14,
wobei der flexible Schlauch dazu ausgelegt ist, für eine medizinische Endoskopvorrichtung verwendet zu werden.

**16.** Medizinische Endoskopvorrichtung aufweisend den flexiblen Schlauch nach einem der Ansprüche 1 bis 15.

**Revendications**

1.  Tube flexible, comprenant :

    un substrat de tube flexible, lequel présente une forme cylindrique et une flexibilité ;
    une couche de résine, laquelle recouvre le substrat de tube flexible, et
    une couche de finition, laquelle recouche la couche de résine,
    dans lequel la couche de finition contient un composé polymère I dérivé de monomères incluant les éléments A à C suivants :

    monomère A : acrylate exempt de groupe hydroxyle présentant un poids moléculaire supérieur ou égal à 110 : 100 parties en masse ;
    monomère B : acrylate exempt de groupe hydroxyle présentant un poids moléculaire supérieur ou égal à 80 et inférieur à 110 : 10 parties en masse à 200 parties en masse, et
    monomère C : acrylate d'hydroxyalkyle : 1 partie en masse à 60 parties en masse.

2.  Tube flexible selon la revendication 1,
    dans lequel le monomère A est de l'acrylate de butyle ou de l'acrylate d'hexyle.

3.  Tube flexible selon la revendication 1 ou 2,
    dans lequel le monomère B est de l'acrylate d'éthyle, de l'acrylate de méthyle, ou du méthacrylate de méthyle.

4.  Tube flexible selon l'une quelconque des revendications 1 à 3,
    dans lequel le monomère C est de l'acrylate d'hydroxyéthyle, de l'acrylate d'hydroxyméthyle, de l'acrylate d'hydroxypropyle, ou de l'acrylate d'hydroxybutyle.

5.  Tube flexible selon l'une quelconque des revendications 1 à 4,
    dans lequel la couche de résine contient au moins un type sélectionné parmi un élastomère d'uréthane, un élastomère de polyuréthane, et un élastomère de polyamide.

6.  Tube flexible selon l'une quelconque des revendications 1 à 5,
    dans lequel la couche de finition est une couche formée en faisant cuire le composé polymère I avec un agent de cuisson.

7.  Tube flexible selon l'une quelconque des revendications 1 à 6,
    dans lequel un module de conservation $E_T'$ du matériau de la couche de finition est inférieur à un module de conservation $E_R'$ du matériau de la couche de résine, et dans lequel le module de conservation est mesuré à 25 °C par le procédé reposant sur JIS-K7244-4.

8.  Tube flexible selon la revendication 7,
    dans lequel le module de conservation $E_T'$ du matériau de la couche de finition est supérieur ou égal à 1 MPa et inférieur ou égal à 20 MPa.

9.  Tube flexible selon la revendication 7 ou 8,
    dans lequel le module de conservation $E_R'$ du matériau de la couche de résine est supérieur ou égal à 1 MPa et inférieur ou égal à 500 MPa.

10. Tube flexible selon la revendication 7 ou 8,
    dans lequel $E_R'$-$E_T'$, laquelle représente une différence entre le module de conservation $E_T'$ du matériau de la couche de finition et le module de conservation $E_R'$ du matériau de la couche de résine, et est supérieure ou égale à 1 MPa et inférieure ou égale à 200 MPa.

11. Tube flexible selon l'une quelconque des revendications 1 à 10,
    dans lequel une épaisseur de la couche de finition est supérieure ou égale à 10 $\mu$m et inférieure ou égale à 200 $\mu$m.

12. Tube flexible selon l'une quelconque des revendications 1 à 11,
    dans lequel la couche de résine présente une couche intérieure et une couche extérieure, et un rapport d'épaisseur entre la couche intérieure et la couche extérieure change avec un gradient dans une direction axiale du substrat de tube flexible.

**13.** Tube flexible selon l'une quelconque des revendications 1 à 12,
dans lequel la couche de résine présente une couche intérieure et une couche extérieure ; un module de conservation de la couche intérieure est supérieur ou égal à 1 MPa et inférieur ou égal à 150 MPa ; un module de conservation de la couche extérieure est supérieur ou égal à 1 MPa et inférieur ou égal à 500 MPa, et une différence obtenue en soustrayant le module de conservation de la couche intérieure du module de conservation de la couche extérieure est supérieure ou égale à 20 MPa et inférieure ou égale à 200 MPa.

**14.** Tube flexible selon la revendication 12,
dans lequel dans une première portion d'extrémité du substrat de tube flexible, le rapport d'épaisseur entre la couche intérieure et la couche extérieure s'étend de 5 : 95 à 40 : 60 (couche intérieure : couche extérieure) ; dans une seconde portion d'extrémité du substrat de tube flexible, le rapport d'épaisseur s'étend de 95 : 5 à 60 : 40 (couche intérieure : couche extérieure), et l'inversion du rapport d'épaisseur se produit entre la première portion d'extrémité et la seconde portion d'extrémité.

**15.** Tube flexible selon l'une quelconque des revendications 1 à 14, dans lequel le tube flexible est configuré pour être utilisé pour un appareil médical endoscopique.

**16.** Appareil médical endoscopique comprenant le tube flexible selon l'une quelconque des revendications 1 à 15.

FIG. 1

FIG. 2

TIP SIDE

REAR -END SIDE

SIDE OF ANGLE PORTION 3b (SOFT)

SIDE OF MAIN BODY OPERATION PORTION 5 (HARD)

## FIG. 3

WEIGHT

θ

## FIG. 4

MANOMETER

CHUCK

SAMPLE

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010239981 A
- JP 2010503436 A
- JP 7039511 A
- JP H07039511 A
- JP 3652138 B
- JP 3542208 B
- JP 3500219 B
- EP 21684714 A
- US 2009069631 A
- JP 2011072391 A